# FASCICULE DE BREVET EUROPEEN

(11) **EP 0 530 084 B1**
(45) Date de publication et mention de la délivrance du brevet: **20.03.1996**
(21) Numéro de dépôt: 92402317.9
(22) Date de dépôt: 20.08.1992
(51) Int. Cl.: A61K 7/48, A61K 7/032

(54) **Composition pour le maquillage et le soin des cils et/ou sourcils et son procédé de préparation**
Wimper- und/oder Augenbraueschminke und Herstellungsverfahren
Eyelash and/or eyebrow make-up composition and preparation method therefor

(30) Priorité: 30.08.1991 FR 9110791
(43) Date de publication de la demande: 03.03.1993
(73) Titulaire: L'OREAL, F-75008 Paris (FR)
(72) Inventeur: Piot, Bertrand, F-92250 La Garenne Colombes (FR); Felardos, Christian, F-75013 Paris (FR); Patraud, Jeanne, Tour Palerme 1216, F-75013 Paris (FR)
(74) Mandataire: Peuscet, Jacques

(56) Documents cités:
- EP-A- 0 332 501
- WO-A-91/12793
- FR-A- 2 085 902
- FR-A- 2 528 699
- GB-A- 2 167 301
- GB-A- 2 207 153
- GB-A- 2 216 797
- PATENT ABSTRACTS OF JAPAN vol. 15, no. 445 (C-884)13 Novembre 1991

## Description

La présente invention concerne une composition cosmétique pour le maquillage et le soin des cils et/ou des sourcils dans laquelle une phase dispersée, à base de cire(s), est répartie à l'intérieur d'une phase dispersante constituée d'une solution aqueuse de polymère.

On sait que les compositions de mascara de type classique sont des émulsions d'au moins une cire dans une phase aqueuse, la stabilité d'une telle émulsion étant assurée par la présence d'un agent tensio-actif comme décrit dans GB-A-2 167 301, GB-A-2 216 797 et GB-A-2 207 15. On sait également que, dans certains cas, l'application de ces compositions sur des sujets ayant les yeux fragiles, entraîne des désagréments de confort et ces désagréments sont généralement imputés à la présence de tensioactifs dans la composition. C'est la raison pour laquelle on a cherché à proposer des compositions de mascara ne contenant pas de tensioactif. Par exemple, dans la demande de brevet JP-60-224610, on a proposé une composition de mascara constituée d'une solution gélifiée de polymères hydrosolubles, dans laquelle sont incorporés des pigments : malheureusement, une telle composition donne un faible résultat de maquillage, notamment en raison de l'absence de cire dans la composition.

Parallèlement aux mascaras classiques, il existe des mascaras waterproof. La qualité primordiale de ces derniers est leur bonne tenue à l'eau, mais leurs qualités de maquillage sont moindres par rapport à un mascara non waterproof.

Leurs compositions sont, soit des dispersions eau dans cires stabilisées par un émulsionnant lipophile, soit des dispersions de cire dans un solvant organique. Dans ce deuxième cas, la présence de tensioactifs n'est pas nécessaire car les cires sont en partie solubilisées par le solvant organique.

De façon plus générale, on a déjà cherché à éviter la présence des tensioactifs dans les produits de maquillage constitués d'une dispersion mais, dans ce cas, la stabilité de la dispersion était difficile à maintenir. On a, par exemple, proposé, dans la demande de brevet JP-02-019310, une composition de maquillage sans tensioactif ; cette composition est une dispersion huile-dans-eau contenant un mélange de poudres hydrophobes et sa stabilité est assurée grâce à l'incorporation, dans sa phase aqueuse, de polymères polyacryliques réticulés. Une telle composition n'est pas destinée au maquillage des cils ou des sourcils et la transposition au domaine spécifique des mascaras ne peut pas logiquement être envisagée pour deux raisons : d'une part, dans le cas d'un mascara, on recherche un chargement important sur les cils alors que, pour les autres produits de maquillage, on recherche au contraire un maquillage estompé ; d'autre part, le maquillage des cils ne peut pas être obtenu de façon satisfaisante en l'absence de cire et les huiles ont une nature et des propriétés physiques très différentes des cires. Il n'était donc pas envisageable pour l'homme de métier de transposer purement et simplement au domaine des mascaras les enseignements du document JP-02-019310.

On a également décrit dans EP-A-0 332 501 une émulsion de type huile dans l'eau contenant dans la phase aqueuse un polyacrylate soluble dans l'eau. Mais les compositions décrites ne contiennent pas plus de 1,5 % de cire en mélange avec des huiles. Ces compositions ne sont pas susceptibles d'être utilisées comme mascara.

Il résulte de cette analyse que, dans l'état de la technique, aucune composition de mascara permettant d'obtenir un maquillage des cils de qualité et bénéficiant d'une bonne stabilité au cours du temps n'avait été proposée sans inclure au moins un tensioactif.

La présente invention a pour but de proposer une composition de mascara pour le maquillage et le soin des cils qui, à la fois, présente une bonne stabilité au cours du temps, fournit un maquillage de qualité et évite toute incorporation de tensioactif dans ladite composition. Pour obtenir un maquillage de qualité, la composition contient, en proportion majeure dans la phase grasse, au moins une cire ; pour obtenir une stabilité satisfaisante pour la composition, qui se présente sous la forme d'une dispersion, on propose une phase dispersante constituée par une solution aqueuse d'au moins un polymère hydrosoluble. Comme indiqué précédemment, il n'était pas évident pour l'homme de métier de pouvoir introduire des cires dans une solution de polymères sans que celles-ci ne coalescent, ce qui aurait entraîné une instabilité de la composition.

Il est rappelé que l'une des caractérisations utilisée pour les cires est la mesure de la "pénétration à l'aiguille". Le principe de cette caractérisation consiste à mesurer la profondeur, exprimée en dixièmes de millimètre, à laquelle pénètre une aiguille normalisée (pesant 2,5 grammes placée dans un porte-aiguille pesant 47,5 grammes) mise en place sur la cire pendant 5 secondes à une température donnée. Dans la présente demande, toute indication relative à la "pénétration d'une aiguille" correspond à une mesure effectuée selon la norme américaine ASTM D5 ou selon la norme française NFT 004 qui est équivalente.

La présente invention a donc pour objet une composition cosmétique liquide ou pâteuse à la température ambiante, destinée au maquillage et au soin des cils et/ou des sourcils et constituée d'une dispersion formée d'une phase dispersante et d'une phase dispersée, la phase dispersante étant une phase aqueuse renfermant au moins un polymère hydrosoluble et la phase dispersée comportant au moins 50% d'une cire ayant un point de fusion compris entre 40 et 110°C et une pénétration à l'aiguille à 25°C comprise entre 3 et 40, la dispersion comprenant au moins 3% de cire et 50% de phase aqueuse.

Les cires utilisées selon l'invention sont, de préférence, choisies dans le groupe formé par les cires animales, les cires végétales, les cires minérales, les cires synthétiques et les fractions diverses de cires naturelles. Parmi les cires animales utilisables, on peut citer les cires d'abeilles, les cires de lanoline et les cires d'insectes de Chine. Parmi les cires végétales, on peut citer la cire de Carnauba, de Candelilla, d'Ouricurry, les cires de fibres de liège, les cires de canne à sucre, les cires du Japon, les cires de jojoba hydrogénées et les huiles hydrogénées qui sont obtenues par hydrogénation catalytique de corps gras composés de chaîne grasse linéaire ou non en C₈ - C₃₂ et qui ont les qualités correspondant à la définition des cires. On peut citer en particulier l'huile de tournesol hydrogénée, l'huile de ricin hydrogénée, l'huile de coprah hydrogénée et la lanoline hydrogénée. Parmi les cires minérales, on peut citer les paraffines, les cires microcristallines, les cires de Montan et les ozokérites. Parmi les cires synthétiques, on peut citer les cires de polyéthylène, les cires obtenues par la synthèse de Fisher et Tropsch, les copolymères cireux ainsi que leurs esters, et les cires de silicone telles que les polyalcoxy- et polyalkylsiloxanes.

Selon l'invention, la composition contient au moins 3 % en poids de cire(s), de préférence 3 à 30 %, par rapport au poids total de la composition.

La composition selon l'invention peut également contenir des charges non colorées ou des pigments colorés.

Les charges, lorsqu'elles sont présentes, sont incluses en une quantité inférieure ou égale à 40% en poids par rapport au poids total de la composition. Lorsque des pigments sont utilisés, ils sont présents dans une proportion inférieure ou égale à 8% en poids par rapport au poids total de la composition.

Les pigments colorés utilisables selon l'invention sont, de préférence, choisis parmi les pigments minéraux, les pigments organiques et les pigments nacrés. A titre d'exemple de pigments minéraux, on peut citer le dioxyde de titane (rutile ou anatase) éventuellement traité en surface et codifié dans le Color Index sous la référence CI 77891, les oxydes de fer noir, jaune, rouge et brun codifiés sous les références CI 77499, 77492 et 77491, le violet de manganèse codifié sous la référence CI 77742, le bleu outremer codifié sous la référence CI 77007, l'oxyde de chrome codifié sous la référence CI 77288, l'hydrate de chrome codifié sous la référence CI 77289 et le bleu ferrique codifié sous la référence CI 77510.

A titre d'exemple de pigments organiques, on peut citer le noir de carbone (carbon black), les pigments D et C red n°19 codifiés dans le Color Index sous la référence CI 45170, les pigments D et C red n° 9 codifiés sous la référence CI 15585, les pigments D et C red n° 21 codifiés sous la référence CI 45380, les pigments D et C orange n° 4 codifiés sous la référence CI 15510, les pigments D et C orange n° 5 codifiés sous la référence CI 45370, les pigments D et C red n° 27 codifiés sous la référence CI 15850, les pigments D et C red n°6 codifiés sous la référence CI 15850, les pigments D et C yellow n°5 codifiés sous la référence CI 45425, les pigments D et C yellow n° 6 codifiés sous la référence CI 15985, les pigments D et C red n°30 codifiés sous la référence CI 73360, les pigments D et C red n°3 codifiés sous la référence CI 45430 et les laques à base de carmin de cochenille codifiées sous la référence CI 75470.

A titre d'exemple de pigments nacrés, on peut citer les pigments nacrés blancs, tels que le mica recouvert d'oxyde de titane et l'oxychlorure de bismuth, les pigments nacrés colorés, tels que le mica-titane avec des oxydes de fer et le mica-titane avec du bleu ferrique ou de l'oxyde de chrome.

On peut avantageusement choisir les charges non colorées dans le groupe formé par : le talc, qui est un silicate de magnésium hydraté, utilisé sous forme de particules généralement de dimensions inférieures à 40 µm (microns) ; les micas, qui sont des aluminosilicates de compositions variées se présentant sous la forme d'écailles ayant des dimensions de 2 à 200 µm (microns), de préférence de 5 à 70 µm (microns), et une épaisseur de 0,1 à 5 µm (microns), de préférence de 0,2 à 3 µm (microns), ces micas pouvant être d'origine naturelle (par exemple muscovite, margarite, roscoelithe, lipidolithe, biotite) ou d'origine synthétique ; l'amidon, en particulier l'amidon de riz ; le kaolin, qui est un silicate d'aluminium hydraté et qui se présente sous la forme de particules de forme isotrope ayant des dimensions généralement inférieures à 30 µm (microns) ; les oxydes de zinc et de titane, généralement utilisés sous la forme de particules ayant des dimensions ne dépassant pas quelques microns (même inférieures à 1 µm (micron) dans le cas de l'oxyde de titane) ; le carbonate de calcium, le carbonate ou l'hydrocarbonate de magnésium ; la cellulose microcristalline ; les poudres de polymères synthétiques, tels que le polyéthylène, les polyesters (par exemple l'isophtalate ou le téréphtalate de polyéthylène), les polyamides (par exemple, celui vendu sous la dénomination commerciale de "nylon"), les "téflons" et les poudres de silicone.

De façon générale, les charges, qu'il s'agisse de pigments colorés ou de charges non colorées, peuvent être enrobées par des substances telles que des acides aminés, des silicones, des sels métalliques ou du collagène, ou avoir subi tout autre traitement permettant de modifier leur état de surface.

Le (ou les) polymère(s) hydrosoluble(s) contenu(s) dans la phase aqueuse est (ou sont) avantageusement choisi(s) dans le groupe formé par les dérivés de protéines d'origine animale ou végétale et, plus particulièrement, les dérivés de kératine, tels que les hydrolysats de kératine et les kératines sulfoniques, les dérivés de chitine ou de chitosanes anioniques, cationiques, amphotères ou non-ioniques, les dérivés de cellulose, tels que l'hydroxyéthylcellulose, l'hydroxypropylcellulose, la méthylcellulose, l'éthylhydroxyéthylcellulose, la carboxyméthylcellulose ainsi que les dérivés quaternisés de la cellulose, les polyéthylèneoxydes, les polypropylèneoxydes, les polymères acryliques, tels que les polyacrylates et les polyméthacrylates,les polyacrylamides et les polyméthacrylamides, ainsi que les copolymères acryliques, les polyvinylpyrrolidones et les copolymères vinyliques, tels que le copolymère de l'éther méthylvinylique et de l'anhydride malique ou le copolymère de l'acétate de vinyle et de l'acide crotonique les polyesters tels que le copolymère diglycol/cyclohexanediméthanol/isophtalates/sulfoisophtalates, les polymères naturels tels que les gommes arabiques, la gomme de guar, les dérivés du xanthane, la gomme de caraya, les alginates, les carraghénates, les glycoaminoglycanes, l'acide hyaluronique et ses dérivés.

Dans la composition selon l'invention, la phase aqueuse réprésente au moins 50% en poids, de préférence de 50 à 97 % en poids par rapport au poids total de la composition. Dans la phase aqueuse, la concentration en polymères(s) hydrosoluble(s) exprimée en poids de manières actives est, de préférence, comprise entre 0,1 et 55% en poids par rapport au poids de la phase aqueuse.

Les compositions selon l'invention peuvent également contenir, en outre, des additifs utilisés, de façon connue, dans les compositions de mascara pour le maquillage ou le soin des cils ou des sourcils et notamment choisies dans le groupe formé par les adoucissants, les conservateurs, les séquestrants, les parfums, les épaississants, les huiles, les silicones, les agents de cohésion, les polymères non hydrosolubles, les agents alcalinisants ou acidifiants et les agents reconnus pour leur action bénéfique sur les cils ou sourcils, tels que les vitamines ou les acides aminés.

La présente invention a également pour objet un procédé de préparation de la composition de maquillage de cils ou de sourcils ci-dessus définie, ce procédé étant caractérisé par le fait que, dans une première étape, on prépare la cire ou le mélange de cires de la phase grasse et on y incorpore éventuellement les additifs liposolubles ; dans une deuxième étape, on mélange à la phase obtenue la (ou les) charge(s) solide(s) sous forme finement divisée ; dans une troisième étape, on prépare une phase aqueuse contenant le (ou les) polymère(s) hydrosoluble(s) et les éventuels additifs éventuellement hydrosolubles ; et, dans une quatrième étape, on ajoute ladite phase aqueuse dans le mélange obtenu à la deuxième étape et on réalise la dispersion de la phase grasse dans la phase aqueuse.

Une autre variante du procédé consiste à mélanger les charges, non pas à la phase cire, mais à la phase aqueuse.

Dans tous les cas, le matériel utilisé pour obtenir une bonne dispersion est celui classiquement utilisé pour l'obtention de mascaras.

Pour mieux faire comprendre l'objet de l'invention, on va donner maintenant, à titre purement illustratif et non limitatif, plusieurs exemples de mise en oeuvre.

Dans les exemples qui suivent, toutes les indications de quantité sont données en grammes.

### EXEMPLE 1 :

On réalise, selon le procédé décrit, la formulation suivante :

| | |
|---|---|
| - Cire de paraffine | 10,0 |
| - Poudre d'amidon | 5,0 |
| - Poudre de polyéthylène vendue sous la dénomination commerciale"ACCUREL EP 400 AKZO" par la société "AKZO" | 5,0 |
| - Poudre d'oxyde de fer noir | 7,0 |
| - Polymère carboxyvinylique vendu sous la dénomination commerciale "CARBOPOL 940" par la société "GOODRICH" | 0,5 |
| - Hydroxyéthylcellulose | 0,5 |
| - Panthénol | 2,0 |
| - Soude qsp pH | 7 |
| - Conservateurs qs | |
| - Eau qsp | 100 |

Cette composition de mascara est stable et permet un maquillage de qualité des cils.

### EXEMPLE 2 :

On réalise, selon le procédé décrit, la formulation suivante :

| | |
|---|---|
| - Cire d'abeilles | 6,0 |
| - Cire de Carnauba | 3,0 |
| - Cire de paraffine | 8,0 |
| - Poudre polymérique vendue sous la dénomination commerciale "ORGASOL 2002" par la société "ATOCHEM" | 5,0 |
| - Copolymère de pyrrolidone anhydre vendu sous la dénomination commerciale "COPOLYMERE 958" par la société "GAF" | 6,0 |
| - Carboxymethylcellulose | 0,4 |
| - Noir de carbone | 2,0 |
| - Conservateur qs | |
| - Eau qsp | 100,0 |

Cette composition de mascara est stable et permet un maquillage de qualité des cils.

### EXEMPLE 3 :

| | |
|---|---|
| - Cire constituée par des huiles végétales hydrogénées vendues sous la dénomination commerciale "SUPPOCIRE" par la société "GATTEFOSSE" | 4,0 |
| - Cire de polyéthylène vendue sous la dénomination commerciale "AC 430" par la société "HOECHST" | 2,0 |
| - Poudre de talc | 10,0 |
| - Poudre d'amidon de riz | 3,0 |
| - Oxyde de fer bleu | 3,0 |
| - Oxyde de fer noir | 5,0 |
| - Polymère vendu sous la dénomination commerciale "JAGUAR C 15" par la société "MEYHALL" | 0,8 |
| - Conservateurs qs | |
| - Eau qsp | 100,0 |

Cette composition de mascara est stable et permet un maquillage de qualité des cils.

### EXEMPLE 4 :

| | |
|---|---|
| - Stéaroxy diméthicone vendue sous la dénomination commerciale "BELSIL SDM 6021 VP 621" par la société "WACKER" | 4,0 |
| - Polyalcoxy (C₁₈-C₃₆) diméthicone vendue sous la dénomination commerciale "176-10877" par la société "GENERAL ELECTRIC" | 12,0 |
| - Cire d'abeilles | 4,0 |
| - Polymère carboxyvinylique vendu sous la dénomination commerciale "CARBOPOL 940" par la société "GOODRICH" | 0,7 |
| - Triéthanolamine | 1,4 |
| - Hydroxyéthylcellulose | 0,6 |
| - Oxyde de fer noir | 7,0 |
| - Conservateur qs | |
| - Eau qsp | 100,0 |

Cette composition de mascara est stable et permet un maquillage de qualité des cils.

## Revendications

1. Composition cosmétique destinée au maquillage ou au soin des cils et/ou des sourcils et constituée d'une dispersion formée d'une phase dispersante et d'une phase dispersée, la phase dispersante étant une phase aqueuse renfermant au moins un polymère hydrosoluble, caractérisée par le fait que ladite phase dispersée contient au moins 50 % d'une cire ayant un point de fusion compris entre 40 et 110°C et une pénétration à l'aiguille à 25°C comprise entre 3 et 40, la dispersion comprenant au moins 3 % de cire et 50 % de phase aqueuse, et ne contient pas d'agent émulsionnant.

2. Composition selon la revendication 1, caractérisée par le fait qu'elle contient de 3% à 30% en poids de cire(s) par rapport au poids total de la composition.

3. Composition selon l'une des revendications 1 ou 2, caractérisée par le fait que la (ou les) cire(s), qu'elle contient, est (ou sont) choisie(s) dans le groupe formé par les cires animales, les cires végétales, les cires minérales, les cires synthétiques et les fractions de cires naturelles.

4. Composition selon la revendication 3, caractérisée par le fait que les cires animales sont choisies dans le groupe formé par les cires d'abeilles, les cires de lanoline et les cires d'insectes de Chine.

5. Composition selon la revendication 3, caractérisée par le fait que les cires végétales sont choisies dans le groupe formé par les cires de Carnauba, de Candelilla, d'Ouricurry, de fibres de liège, de canne à sucre et du Japon, les cires de jojoba hydrogénées et les huiles hydrogénées.

6. Composition selon la revendication 3, caractérisée par le fait que les cires minérales sont choisies dans le groupe formé par les cires de paraffine, les cires microcristallines, les cires de Montan et les ozokérites.

7. Composition selon la revendication 3, caractérisée par le fait que les cires synthétiques sont choisies dans le groupe formé par les cires de polyéthylène, les cires obtenues par la synthèse de Fisher et Tropsch, les cires de silicone et les copolymères cireux ainsi que leurs esters.

8. Composition selon l'une des revendications 1 à 7, caractérisée par le fait qu'elle contient jusqu'à 40 % en poids de charge(s) par rapport au poids total de la composition.

9. Composition selon la revendication 8, caractérisée par le fait que la (ou les) charges(s) qu'elle contient est (ou sont) choisie(s) dans le groupe formé par les charges non colorées et les pigments colorés.

10. Composition selon la revendication 9, caractérisée par le fait qu'elle contient jusqu'à 8 % en poids de pigments colorés.

11. Composition selon la revendication 10, caractérisée par le fait qu'elle contient au moins un pigment coloré pris dans le groupe formé par les pigments minéraux, les pigments organiques et les pigments nacrés.

12. Composition selon la revendication 9, caractérisée par le fait qu'elle contient au moins une charge non colorée prise dans le groupe formé par le talc, les micas, l'amidon, le kaolin, l'oxyde de zinc, l'oxyde de titane, le carbonate de calcium, le carbonate ou l'hydrocarbonate de magnésium, la cellulose microcristalline et les poudres de polymères synthétiques.

13. Composition selon l'une des revendications 1 à 12, caractérisée par le fait qu'elle contient de 50 à 97 % en poids de phase aqueuse par rapport au poids total de la composition.

14. Composition selon l'une des revendications 1 à 13, caractérisée par le fait que sa phase aqueuse contient de 0,1 à 55 % en poids de matières actives d'au moins un polymère hydrosoluble, ce pourcentage étant donné par rapport au poids total de la dite phase aqueuse.

15. Composition selon l'une des revendications 1 à 14, caractérisée par le fait qu'elle contient au moins un polymère hydrosoluble choisi dans le groupe formé par les dérivés de protéines animales ou végétales, les dérivés de chitine ou de chitosane, les dérivés de cellulose, les polymères ou copolymères acryliques, les polyvinylpyrrolidones, les copolymères vinyliques, les polyesters, les polyéthylèneoxydes, les polypropylèneoxydes et les polymères naturels.

16. Composition selon l'une des revendications 1 à 15, caractérisée par le fait qu'elle contient au moins un additif pris dans le groupe formé par les adoucissants, les conservateurs, les séquestrants, les parfums, les épaississants, les huiles, les silicones, les agents de cohésion, les polymères non hydrosolubles, les agents alcalinisants ou acidifiants, et les agents reconnus pour leur action bénéfique sur les cils ou les sourcils.

17. Procédé de préparation d'une composition de maquillage ou de soin pour les cils et/ou les sourcils selon l'une des revendications 1 à 16, caractérisé par le fait que, dans une première étape, on prépare la cire ou le mélange de cires de la phase grasse et on y incorpore éventuellement le (ou les) additif(s) liposoluble(s) de la composition ; dans une deuxième étape, on mélange à la phase obtenue la (ou les) charge(s) solide(s) sous forme finement divisée ; dans une troisième étape, on prépare une phase aqueuse contenant le (ou les) polymère(s) hydrosoluble(s) et le (ou les) éventuel(s) additif(s) hydrosoluble(s) ; et, dans une quatrième étape, on ajoute ladite phase aqueuse dans le mélange obtenu à la deuxième étape et on réalise la dispersion de la phase grasse dans la phase aqueuse.

18. Procédé de préparation d'une composition de maquillage ou de soin des cils et/ou des sourcils selon l'une des revendications 1 à 16, caractérisé par le fait que, dans une première étape, on prépare la cire ou le mélange de cires de la phase grasse et on y incorpore éventuellement le (ou les) additif(s) liposoluble(s) de la composition ; dans une deuxième étape, on prépare une phase aqueuse contenant le (ou les) polymère(s) hydrosoluble(s) et le (ou les) éventuel(s) additif(s) hydrosoluble(s) ; dans une troisième étape, on ajoute à la phase aqueuse obtenue, la (ou les) charge(s), et dans une quatrième étape, on ajoute la phase aqueuse dans la phase de cire et on réalise la dispersion de la phase grasse dans la phase aqueuse.

## Claims

1. Cosmetic composition intended for the make-up or care of the eyelashes and/or eyebrows and consisting of a dispersion made up of a dispersant phase and of a disperse phase, the dispersant phase being an aqueous phase containing at least one water-soluble polymer, characterized in that the said disperse phase contains at least 50 % of a wax which has a melting point of between 40 and 110°C and a needle penetration at 25°C of between 3 and 40, the dispersion comprising at least 3 % of wax and 50 % of aqueous phase, and does not contain any emulsifying agent.

2. Composition according to Claim 1, characterized in that it contains from 3 % to 30 % by weight of wax(es) relative to the total weight of the composition.

3. Composition according to either of Claims 1 and 2, characterized in that the wax(es) which it contains is (or are) chosen from the group made up of animal waxes, vegetable waxes, mineral waxes, synthetic waxes and fractions of natural waxes.

4. Composition according to Claim 3, characterized in that the animal waxes are chosen from the group made up of beeswaxes, lanolin waxes and Chinese insect waxes.

5. Composition according to Claim 3, characterized in that the vegetable waxes are chosen from the group made up of carnauba, candelilla, ouricury, cork fibre, sugar cane and Japan waxes, hydrogenated jojoba waxes and hydrogenated oils.

6. Composition according to Claim 3, characterized in that the mineral waxes are chosen from the group made up of paraffin waxes, microcrystalline waxes, montan waxes and ozokerites.

7. Composition according to Claim 3, characterized in that the synthetic waxes are chosen from the group made up of polyethylene waxes, the waxes obtained by the Fischer-Tropsch synthesis, silicone waxes and waxy copolymers as well as their esters.

8. Composition according to one of Claims 1 to 7, characterized in that it contains up to 40 % by weight of filler(s) relative to the total weight of the composition.

9. Composition according to Claim 8, characterized in that the filler(s) which it contains in (or are) chosen from the group made up of colourless fillers and coloured pigments.

10. Composition according to Claim 9, characterized in that it contains up to 8 % by weight of coloured pigments.

11. Composition according to Claim 10, characterized in that it contains at least one coloured pigment taken from the group made up of inorganic pigments, organic pigments and pearlescent pigments.

12. Composition according to Claim 9, characterized in that it contains at least one colourless filler taken from the group made up of talc, micas, starch, kaolin, zinc oxide, titanium oxide, calcium carbonate, magnesium carbonate or hydrocarbonate, microcrystalline cellulose and synthetic polymer powders.

13. Composition according to one of Claims 1 to 12, characterized in that it contains from 50 to 97 % by weight of aqueous phase relative to the total weight of the composition.

14. Composition according to one of Claims 1 to 13, characterized in that its aqueous phase contains from 0.1 to 55 % by weight of active materials of at least one water-soluble polymer, this percentage being given in relation to the total weight of the said aqueous phase.

15. Composition according to one of Claims 1 to 14, characterized in that it contains at least one water-soluble polymer chosen from the group made up of the animal or vegetable protein derivatives, chitin or chitosan derivatives, cellulose derivatives, acrylic polymers or copolymers, polyvinylpyrrolidones, vinyl copolymers, polyesters, polyethylene oxides, polypropylene oxides and natural polymers.

16. Composition according to one of Claims 1 to 15, characterized in that it contains at least one additive taken from the group made up of emollients, preserving agents, sequestrants, perfumes, thickeners, oils, silicones, cohesion agents, water-insoluble polymers, alkalifying or acidifying agents and agents recognized for their beneficial effect on eyelashes or eyebrows.

17. Process for the preparation of a composition for make-up or care for the eyelashes and/or eyebrows, according to one of Claims 1 to 16, characterized in that, in a first stage, the wax or the mixture of waxes of the fatty phase is prepared and the liposoluble additive(s) of the composition is (or are) optionally incorporated into it, in a second state the solid filler(s) in finely divided form is (or are) mixed with the phase obtained; in a third stage an aqueous phase containing the water-soluble polymer(s) and the optional water-soluble additive(s) is prepared; and, in a fourth stage, the said aqueous phase is added to the mixture obtained in the second stage and the dispersion of the fatty phase in the aqueous phase is produced.

18. Process for the preparation of a composition for make-up or care of the eyelashes and/or eyebrows, according to one of Claims 1 to 16, characterized in that, in a first stage, the wax or the mixture of waxes of the fatty phase is prepared and the liposoluble additive(s) of the composition is (or are) optionally incorporated into it; in a second stage an aqueous phase containing the water-soluble polymer(s) and the optional water-soluble additive(s) is prepared, in a third stage the filler(s) is (or are) added to the aqueous phase obtained and, in a fourth stage, the aqueous phase is added to the wax phase and the dispersion of the fatty phase in the aqueous phase is produced.

## Patentansprüche

1. Kosmetisches Mittel zum Schminken oder zur Pflege der Wimpern und/oder der Augenbrauen, welches aus einer Dispersion einer dispergierenden Phase und einer dispergierten Phase gebildet wird, wobei die dispergierende Phase eine wäßrige Phase ist, die wenigstens ein wasserlösliches Polymer umfaßt, dadurch gekennzeichnet, daß die dispergierte Phase wenigstens 50 % eines Wachses mit einem Schmelzpunkt von 40 bis 110° C und einer Nadelpenetration bei 25° C von 3 bis 40 enthält, wobei die Dispersion wenigstens 3 % Wachs und 50 % wäßrige Phase umfaßt, und kein Emulsionsmittel enthält.

2. Mittel nach Anspruch 1, dadurch gekennzeichnet, daß es 3 Gew.-% bis 30 Gew.-% Wachs(e), bezogen auf das Gesamtgewicht des Mittels, enthält.

3. Mittel nach einem der Ansprüche 1 oder 2, dadurch gekennzeichnet, daß das (oder die) Wachs(e), das (oder die) es enthält, ausgewählt ist (oder Sind) unter tierischen Wachsen, pflanzlichen Wachsen, anorganischen Wachsen, synthetischen Wachsen und Fraktionen natürlicher Wachse.

4. Mittel nach Anspruch 3, dadurch gekennzeichnet, daß die tierischen Wachse ausgewählt sind unter Bienenwachsen, Lanolinwachsen und Chinawachsen.

5. Mittel nach Anspruch 3, dadurch gekennzeichnet, daß die pflanzlichen Wachse ausgewählt sind unter Carnauba-, Candelilla-, Ouricury-, Eichenfaser-, Zuckerrohr- und Japanwachsen, Wachsen aus hydrogeniertem Jojoba und hydrogenierten Ölen.

6. Mittel nach Anspruch 3, dadurch gekennzeichnet, daß die anorganischen Wachse ausgewählt sind unter Paraffinwachsen, mikrokristallinen Wachsen, Montanwachsen und Ozokeriten.

7. Mittel nach Anspruch 3, dadurch gekennzeichnet, daß die synthetischen Wachse ausgewählt sind unter Polyethylenwachsen, durch die Fischer-Tropsch-Synthese erhaltenen Wachsen, Silikonwachsen und wachsartigen Copolymeren, sowie Estern davon.

8. Mittel nach einem der Ansprüche 1 bis 7, dadurch gekennzeichnet, daß es bis zu 40 Gew.-% Füllstoff(e), bezogen auf das Gesamtgewicht des Mittels, enthält.

9. Mittel nach Anspruch 8, dadurch gekennzeichnet, daß der (oder die) Füllstoff(e), den (oder die) es enthält, ausgewählt ist (oder sind) unter farblosen Füllstoffen und Farbpigmenten.

10. Mittel nach Anspruch 9, dadurch gekennzeichnet, daß es bis zu 8 Gew.-% Farbpigmente enthält.

11. Mittel nach Anspruch 10, dadurch gekennzeichnet, daß es wenigstens ein Farbpigment enthält, das ausgewählt ist unter anorganischen Pigmenten, organischen Pigmenten und perlmutterartiger Pigmenten.

12. Mittel nach Anspruch 9, dadurch gekennzeichnet, daß es wenigstens einen farblosen Füllstoff enthält, welcher ausgewählt ist unter Talk, Glimmer, Stärke, Kaolin, Zinkoxid, Titanoxid, Calciumcarbonat, Magnesiumcarbonat oder -hydrogencarbonat, mikrokristalliner Cellulose und Pulvern synthetischer Polymere.

13. Mittel nach einem der Ansprüche 1 bis 12, dadurch gekennzeichnet, daß es 50 bis 97 Gew.-% wäßrige Phase, bezogen auf das Gesamtgewicht des Mittels, enthält.

14. Mittel nach einem der Ansprüche 1 bis 13, dadurch gekennzeichnet, daß seine wäßrige Phase 0,1 bis 55 Gew.-% aktives Material wenigstens eines wasserlöslichen Polymers enthält, wobei dieser Prozentsatz auf das Gesamtgewicht der wäßrigen Phase bezogen ist.

15. Mittel nach einem der Ansprüche 1 bis 14, dadurch gekennzeichnet, daß es wenigstens ein Wasserlösliches Polymer enthält, das ausgewählt ist unter Derivaten tierischer oder pflanzlicher Proteine, Chitin- oder Chitosanderivaten, Cellulosederivaten, Acrylpolymeren oder -copolymeren - Polyvinylpyrrolidonen, Vinylcopolymeren, Polyestern, Polyethylenoxiden, Polypropylenoxiden und natürlichen Polymeren.

16. Mittel nach einem der Ansprüche 1 bis 15, dadurch gekennzeichnet, daß es wenigstens ein Additiv enthält, das ausgewählt ist unter Weichmachern, Konservierungsmitteln, Sequestriermitteln, Parfümen, Dickungsmitteln, Ölen, Silikonen, Kohäsionsmitteln, wasserunlöslichen Polymeren, Alkalisierungs- oder Ansäuerungsmitteln, und Mitteln, die für ihre vorteilhafte Wirkung auf die Wimpern oder die Augenbrauen bekannt sind.

17. Verfahren zur Herstellung eines Mittels zum Schminken oder zur Pflege der Wimpern und/oder der Augenbrauen nach einem der Ansprüche 1 bis 16, dadurch gekennzeichnet, daß man in einem ersten Schritt das Wachs oder die Wachsmischung der Fettphase herstellt und dazu gegebenenfalls das (oder die) fettlösliche(n) Additiv(e) des Mittels gibt; in einem zweiten Schritt der erhaltenen Phase den (oder die) festen Füllstoff(e) in fein verteiltem Zustand beimischt; in einem dritten Schritt eine wäßrige Phase herstellt, die das (oder die) wasserlösliche(n) Polymer(e) und gegebenenfalls das (oder die) wasserlösliche(n) Additiv(e) enthält; und in einem vierten Schritt die wäßrige Phase in die im zweiten Schritt erhaltene Mischung gibt und die Dispersion der Fettphase in der wäßrigen Phase herstellt.

18. Verfahren zur Herstellung eines Mittels zum Schminken oder zur Pflege der Wimpern und/oder der Augenbrauen nach einem der Ansprüche 1 bis 16, dadurch gekennzeichnet, daß man in einem ersten Schritt das Wachs oder die Wachsmischung der Fettphase herstellt, und dazu gegebenenfalls das (oder die) fettlösliche(n) Additiv(e) des Mittels gibt; in einem zweiten Schritt eine wäßrige Phase herstellt, welche das (oder die) wasserlösliche(n) Polymer(e) und gegebenenfalls das (oder die) wasserlösliche(n) Additiv(e) enthält; in einem dritten Schritt zu der erhaltenen wäßrigen Phase den (oder die) Füllstoff(e) gibt, und in einem vierten Schritt die wäßrige Phase in die Wachsphase gibt und die Dispersion der Fattphase in der wäßrigen Phase herstellt.
